# EUROPEAN PATENT APPLICATION

(11) **EP 2 044 929 A1**
(43) Date of publication of application: **08.04.2009**
(21) Application number: 07380269.6
(22) Date of filing: 04.10.2007
(51) Int. Cl.: A61K 9/00, A61K 9/50, A61K 31/4439, A61K 47/10

(54) **Oral fast distintegrating tablets**

(71) Applicant: Laboratorios del Dr. Esteve S.A., 08041 Barcelona (ES)
(72) Inventor: Soler Ranzani, Luis, 08013 Barcelona (ES); Casadevall Pujals, Gemma, 08019 Barcelona (ES)
(74) Representative: ABG Patentes, S.L.

(57) **Abstract**

The present invention refers to an orally fast disintegrable pharmaceutically acceptable multiple units tablet dosage form comprising a) tablet excipients comprising a disintegrant and b) individual units wherein each individual unit comprises:
i) a core material comprising at least one physiologically active substance;
ii) a controlled release coating layer; and
iii) an over-coating layer comprising a mixture of plasticizer agents.

## Description

### FIELD OF THE INVENTION

The present invention relates to the field of multi (micro) particulate pharmaceutical compositions, particularly, orally fast disintegrable pharmaceutically acceptable multiple units tablet dosage forms that allow the release of at least one medicinal and/or nutritional active substance in vivo.

### BACKGROUND OF THE INVENTION

Pharmaceutical solid dosage forms, for example, tablets, are usually prepared to deliver pharmaceutically active substances in a digestive organ by disintegration or dissolution through oral administration, without fast disintegration or dissolution in the oral cavity.

In view of the aging population and their changes in life environment, it is desirable to develop an orally disintegrable solid dosage form capable of being administered without water, retaining the convenience for use which is a characteristic of a tablet, and being administered on demand easily, anytime and anywhere, without water.

For many reasons, such as, masking a bitter taste, or providing enteric abilities or release abilities, it is desirable to prepare the solid pharmaceutical dosage forms as granules (or fine granules). Solid granules or fine granules in which the active substance of the drug is enterically coated sometimes require coating of the whole surface of the particle, including the enteric coating.

In K. Lehman et al. Drugs made in German (1994), 37, N° 2, 53-60, small particles such as crystals, granules and pellets of a particle size in the range of 0,3-1,2 mm were coated with aqueous dispersions of methacrylic acid and methacrylic ester copolymers (Eudragit RL 30D, RS 30D, L 30 D-55 and NE 30 D) to provide taste masking, resistance to gastric fluid and sustained release properties; and then compressed into fast disintegrating tablets. Damage in the coatings was observed with brittle coating materials when elongation at break was around 20%. More flexible films were observed for uncompressed particles.

WO 99/59544 describes an orally disintegrable tablet (ODT) which comprises (i) fine granules having an average particle diameter of 400 µm or less, which fine granules comprises a layer, made of a water soluble sugar-alcohol, preferably mannitol, coating the plasticized enteric layer which may be constructed by several layers of plasticized enteric coatings.

International patent application WO 96/01624 refers to a pharmaceutical multiple unit tablet dosage form comprising an acid labile H+K+-ATPase inhibitor, in which the active substance is in the form of individually enteric coating layered units compressed into a tablet. The enteric coating layer(s) covering the individual units of active substance are plasticized, allowing that the compression of the units into a tablet does not significantly affect the acid resistance of the individually enteric coating layered units.

Patent US 5464632 describes a rapidly disintegratable multiparticulate tablet, characterized by the fact that the active substance is present in the form of coated microcrystals or microgranules and that the compression base comprises at least one disintegrant and at least one swelling-agent.

WO 03/077888 discloses orally dispersible tablets based on microcapsules of the reservoir type that allow the prolonged releases of at least one medicinal and/or nutritional active in vivo, wherein each capsule comprises a hard, i.e. mechanically non-deformable, core of active substance (crystals, granules). These microcapsules comprise at least one outer over-coating envelope based on at least one deformable organic constituent having a melting point of between 40 °C and 120 °C, and preferably of between 45 °C and 110 °C. The outer over-coating envelope belonging to the microcapsules used in the tablet results from a choice of deformable constituent from organic compounds having a melting point of between 40 and 120 °C, these compounds being selected:
- from polymers and copolymers based on alkylene glycols, particular preference being given to polyethylene glycols (PEGs) and especially to polyethylene glycols having a molecular weight of 6,000 to 20,000 D, and/or
- from fats such as hydrogenated vegetable oils, fatty acids, fatty alcohols, fatty acid and/or fatty alcohol esters, polyolefins and mineral, vegetable, animal or synthetic waxes, particular preference being given according to the invention to fatty acid esters such as diglycerides and triglycerides and mixtures thereof, glycerol behenate and hydrogenated oils, hydrogenated castor, soya bean, cottonseed and palm oils.

Said organic over-coating constituent(s) with a melting point between 40 and 120 °C can be used individually or in a mixture thereof, and optionally in association with: a mineral filler, for example silica or titanium dioxide, or an organic filler, for example microcrystalline cellulose, and/or at least one lubricant, for example magnesium stearate or sodium benzoate, and/or at least one hydrophilic polymer such as water-soluble cellulose derivatives like hydroxypropyl cellulose or hydroxypropyl methyl cellulose, synthetic polymers such as polyvinylpyrrolidone or polyvinyl alcohols (PVA), or acrylic and methacrylic derivatives, for example EUDRAGIT(R), and/or a surfactant, for example a polyethoxylated sorbitan(R) ester.

This overview of the state of the art shows that it is difficult to find a technical compromise that guarantees both, the stability of the release profile of the active substance in a multiparticulate modified release ODT dosage form and the maintenance of the properties of the microcapsules thereof.

For example, it is difficult to find the correct hardness which maintains the mechanical and release properties of the "compressed" microcapsules once tabletted with respect of the "uncompresssed" microcapsules.

There is also a need in the art for providing modified release multiple units tablet dosage forms having high hardness and which at the same time readily disintegrate in the mouth. The modified release multiple units tablet dosage forms must be also capable of providing good organoleptic properties (flavour masking, smooth feeling). These properties are important for providing formulations with improved patient acceptance.

Thus, there is still a need to find an orally fast disintegrable pharmaceutical multiple units tablet dosage form that avoids the drawbacks of those known. A desirable approach to the problem would be to propose a simple technical solution adaptable to any microcapsule or microsphere.

### BRIEF DESCRIPTION OF THE INVENTION

As a result of their research the inventors have developed an orally fast disintegrable pharmaceutical multiple units tablet dosage form with excellent and repeatable release profile of the active substance. Said dosage forms readily disintegrate in the mouth and overcome the drawbacks of known ODTs.

According to one aspect, the invention is directed to an orally fast disintegrable pharmaceutically acceptable multiple units tablet dosage form (also referred to as "disintegrable dosage form of the invention") comprising a) tablet excipients comprising a disintegrant and b) individual units wherein each individual unit comprises:
i) a core material comprising at least one physiologically active substance;
ii) a controlled release coating layer; and
iii) an over-coating layer comprising a mixture of plasticizer agents.

The units that constitute the tablet dosage form have an excellent performance during the compression process necessary for their manufacture. Due to their good compressibility, it is possible to obtain a high ratio of units in the tablet and consequently, a higher amount of active substance in a tablet of a given size.

Also, the new tablets can be compressed to high hardness, at least up to 8.5 Kp, and are not unit size dependent; pellets up to 0.8 mm were compressed having the above mentioned characteristics.

Further, the disintegrable dosage forms of the invention achieve the difficult balance between a high enough hardness of the tablets and short disintegrating times.

The disintegrable dosage forms of the invention also have good patient acceptance, providing dosage forms which effectively mask unpleasant flavours, and have a smooth feeling in the mouth.

### DETAILED DESCRIPTION OF THE INVENTION

By the term "fast disintegrable pharmaceutically acceptable" it is understood a preparation in solid state which comprises a pharmaceutically active substance and a disintegrant excipient mixture, which upon contacting to an appropriate medium, such as saliva, gastric fluid, water, soap, milk, etc... is efficiently disintegrated.

The disintegrable dosage form of the invention can be administered without water or together with water, e.g. by dissolution or disintegration together with a little water, or without water, e.g. with saliva, in the oral cavity, before swallowing. Alternatively, the disintegrable dosage form of the invention may be administered with water, and swallowed without prior dissolution or disintegration. Also the solid preparation may be administered pre-dissolved or pre-disintegrated in water.

Preferably, the pharmaceutical dosage form of the invention is administered without water, e.g. when the patient has difficulty in swallowing tablets, or for administration to the aged or to children when there is a fear of blocking the throat if a non orally disintegrable tablet is used. In a particular embodiment, the individual units which comprise the pharmaceutically active substance are in the form of fine granules, more preferably the fine granules are pellets. By the term "fine granule" it is understood a granule, pellet, bead, spheroid, among others, in the particle size range from 100 to 2500 µm.

The disintegrable dosage form of the invention can be safely administered orally to mammals, specially humans.

By the term 'physiologically active substance' it is understood a pharmaceutically active substance, a flavour ingredient or a nutritional ingredient.

As pharmaceutically active substance, for example, one or more substances selected from the group consisting of gastrointestinal function conditioning agents, anti-inflammatory agents, analgesics, anti-migraines, antihistaminic agents, cardiovascular agents, diuretics, anti-hypertensive agents, anti-hypolipidemic agents, anti-ulcer agents, anti-emetics agents, anti-asthmatic agents, anti-depressants, vitamins, anti-thrombic agents, chemotherapeutic agents, hormones, anthelmintic agents, anti-diabetic agents, anti-viral agents and mixtures thereof can be used.

Representative examples of the above-mentioned gastrointestinal function conditioning agents include bromopride, metoclopramide, cisapride and domperidone; the anti-inflammatory agents, aceclofenac, diclofenac, flubiprofen, sulindac and celecoxib; the analgesics, acetaminophen, ibuprofen and aspirin; the anti-migraines, sumatriptan and ergotamine; the antihistaminic agents, loratadine, fexofenadine and cetirizine, the cardiovascular agents, nitroglycerine, and isosorbide dinitrate; the diuretics, furocemide and spironolactone; the anti-hypertensive agents, propanolol, amlodipine, felodipine, captoprile, ramiprile, losartan, valsartan, eprosartan, irbesartan, tasosartan, telmisartan; the anti-hypolipidemic agents, simvastatin, atorvastatin and pravastatin; the anti-ulcer agents, cimetidine, ranitidine, famotidine, lansoprazole, omeprazole, rabeprazole and pantoprazole; the antiemetics, meclizine hydrochloride, ondansetron, granisetron, ramosetron and tropisetron; the anti-asthmatic agents, aminophylline, theophylline, terbutaline, fenoterol, formoterol and ketotifen; the anti-depressants, fluoxetine and sertraline; the anti-thrombotic agents, sulfinpyrazone, dipyridamole and ticlopidine; the chemotherapeutic agents, cefaclor, bacampicillin, sulfamethoxazole and rifampicin; the hormones, dexamethasone and methyltestosterone; the anthelmintic agents, pieperazine, ivermectine and mebendazole; and the anti-diabetic agents, acarbose, gliclazid and glipizid.

According to a preferred embodiment, the pharmaceutically active substance is an acid labile substance. Preferably, said acid-labile substance is mixed with alkaline compounds. It is difficult to compress enterically coated pellets maintaining their characteristics. Acid labile substances require enteric coating since contact with acidic media such as the stomach should be avoided. In such cases, effectively coating the enteric layer against acidic conditions becomes essential to obtain a suitable *"in vivo"* delivery of the active substance.

In a particular embodiment of the invention, the pharmaceutically active substance is an anti-ulcer agent or a H+/K+-ATPase inhibitor, preferably is a benzimidazole derivative or one of its single enantiomers or a salt thereof, more preferably is lansoprazole, omeprazole, rabeprazole or pantoprazole, even more preferably is lansoprazole

In another particular embodiment, the pharmaceutically active substance is a non-steroidal anti-inflammatory drug or a salt thereof, more preferably is aspirin.

The nutritional ingredient which is included in the solid dosage form can be selected from the group consisting of vitamins, such as vitamin A, vitamin D, vitamin E (d-alphatocopherol acetic acid), vitamin B1 (dibenzoyl thiamine, fursultiamine hydrochloride), vitamin B2 (riboflavin tetrabutyrate), vitamin B6 (pyridoxine hydrochloride), vitamin C (ascorbic acid, sodium L-ascorbate) and vitamin B12 (hydroxocobalamin acetate); minerals such as calcium, magnesium and iron; proteins; amino acids; oligosaccharides, unsaturated fatty acids, herbs and mixtures thereof.

By the term 'tablet excipients' it is understood a mixture of a disintegrant and other physiologically acceptable additives or excipients which are used in the elaboration of disintegrable tablets. In addition to disintegrants, the excipients may be selected form the group consisting of binders, foaming agents, sweeteners, fillers, flavouring agents, lubricants, masking agents, colorants, stabilizers, diluting agents and mixtures thereof. By the term 'disintegrant' it is understood a substance which, upon addition to a solid preparation, facilitates its break-up or disintegration after administration and permits the release of an active substance as efficiently as possible to allow for its rapid dissolution.

As examples of the disintegrating agent, starches such as corn starch and potato starch, partial alpha starch, sodium carboxymethyl starch, carmellose, carmellose calcium, crosscarmellose sodium, polyvinyl alcohol, crospovidone, low-substituted hydroxypropyl cellulose, crystalline cellulose, hydroxypropyl starch and the like can be given.

As examples of the binder, hydroxypropyl methyl cellulose, carboxyvinyl polymer, carmellose sodium, alpha starch, polyvinylpyrrolidone, gum Arabic, gelatin, pullulan and the like can be given.

As examples of filler, sucrose, glucose, lactose, mannitol, xylitol, dextrose, microcrystalline cellulose, maltose, sorbitol, calcium phosphate, calcium sulphate and the like can be given.

As examples of the foaming agent, sodium bicarbonate can be used.

As examples of the sweetener, sodium saccharin, dipotassium glycyrrhizin, aspartame, stevia, thaumatin and the like can be given.

As examples of the masking agent, water insoluble polymers such as ethyl cellulose, polymers insoluble in saliva and soluble in gastric fluid such as a copolymer of methyl methacrylate, butyl methacrylate, and diethylaminoethyl methacrylate, and the like can be given.

As examples of flavouring, perfume, lemon, lemon-lime, orange, menthol, peppermint oil, vanillin or powders of these absorbed with dextrin or cyclodextrin, and the like can be used.

As examples of the lubricant, magnesium stearate, magnesium stearate, fumarate stearyl, talc, stearic acid, colloidal silicon dioxide (Aerosil 200®) and the like can be given.

As examples of the colorant, food dyes such as food yellow No. 5, food red No. 3, food blue No. 2, food lake dye, red iron oxide, and the like can be given.

As examples of the stabilizer or solubilizer, antioxidants such as ascorbic acid and tocopherol, surfactants such as polysorbate 80 and the like can be given depending on the physiologically active substance used.

According to a preferred embodiment, the controlled release coating layer is an enteric coating layer. Of course, the disintegrable dosage form of the invention may comprise additional layers. Thus, according to a preferred embodiment, the disintegrable dosage form of the invention further comprises one or more separating layer(s) over the core material and below the controlled release coating layer.

The disintegrable dosage form of the invention comprises an over-coating layer comprising a mixture of plasticizer agents. For the purposes of the invention, a plasticizer is a substance that is normally used to improve the mechanical properties of a film formed by a polymeric substance. It is a product which does not return to its original form after deformation. When added to a polymeric substance, plasticizers provide a material with improved resistance and flexibility. For the purposes of the present invention, plasticizers are preferably solid at room temperature and water soluble.

Thus, preferably, at least one of the plasticizer agents is selected from the group consisting of, a wax, linoline-type alcohols, a gelatine, a polyethylene glycol, a polypropylene glycol, triacetin, tributyl citrate, dibutyl sebacate, medium chain length triglyceride fatty acids, resin acid, long chain fatty acids (e.g. stearic acid, palmitic acid) or mixtures thereof.

Other suitable plasticizer agents are those selected from the group consisting of gliceryl monostearate, stearic acid, glyceryl palmine stearate, glyceryl dibehenate and the like.

According to a preferred embodiment, the over-coating layer of the invention comprises a first polyethylene glycol and a second plasticizer agent, which is more preferably a second polyethylene glycol.

According to a preferred embodiment, the average molecular weight of said first polyethylene glycol is lower than 6000.

According to a further preferred embodiment, the over-coating layer comprises a mixture of polyethylene glycol 4000 and polyethylene glycol 6000 as plasticizer agents.

The best results have been obtained with a mixture of PEG having different viscosity and molecular weigh. According to a preferred embodiment, the over-coating layer comprises a mixture of polyethylene glycol 4000, polyethylene glycol 6000 and polyethylene glycol 8000.

According to a preferred embodiment, the over-coating layer comprises more than 80 % w/w of a plasticizer mixture, preferably more than 90% w/w of a plasticizer mixture, more preferably more than 95% w/w of a plasticizer mixture.

The disintegrable dosage form of the invention can be obtained by procedures which are well-known in the art. For example, the excipient mixture (e.g. compression base) and the individual units which comprise the pharmaceutically active substance can be uniformly mixed together and then subjected to compression to provide a solid preparation in the form of a tablet.

The individual units which comprise the pharmaceutically active substance can be produced by a known granulation method. This method includes, for example, rolling granulation method, fluidized-bed granulation, stirring granulation and the like. The granules can contain a core together with or separately from the physiologically active substance. The core is optionally coated with the physiologically active substance and further coated for masking taste or smell and/or for imparting enteric dissolubility by known methods, for example, by a method which comprises coating a core comprising crystalline cellulose and lactose with an acid-labile physiologically active substance and then further with other excipients as binders, lubricants, water-soluble polymers, etc...

In a particular embodiment of the invention, the individual units which comprises the pharmaceutically active substance are pelletized, for example by conventional extrusion, hot melt granulation, hot melt extrusion, roller compaction, compaction or layering processes, and then mixed with the compression base. In a preferred embodiment of the invention said pellets are prepared by an aqueous layering process.

The mixture of the compression base and the individual units which comprises the pharmaceutically active substance is then fed to the die of a tablet press and sufficient pressure is applied to form a solid tablet. Such pressure can vary, and typically ranges about 1.000 - 20.000 N, being particularly preferable 3.000 - 15.000 N. The resulting compressed solid preparation possesses a suitable strength and hardness and does not disintegrate during distribution and storage.

In the particular case when a benzimidazole compound or a salt thereof such as lansoprazole is employed as an acid-labile pharmaceutical active substance, the orally disintegrable tablet of the present invention comprises enteric fine granules. This formulation is useful for the treatment and prevention of digestive ulcer (gastric ulcer, duodenal ulcer, anastomotic ulcer, etc...), gastritis, reflux esophagitis, eradication of H. *pylori*, suppression of gastrointestinal bleeding caused by digestive ulcer, acute stress ulcer and hemorrhagic gastritis, suppression of gastrointestinal bleeding caused by invasive stress, treatment and prevention of ulcer caused by non-steroidal anti-inflammatory agent, treatment and prevention of gastric hyperacidity and ulcer caused by postoperative stress. The dosage of the preparation per an adult is about 0.5 to 1.500 mg/day, preferably about 5 to 150 mg/day, as a benzimidazole compound or a salt thereof.

The disintegrable dosage form of the invention thus obtained exhibits disintegrable or dissolubility in the oral cavity, aqueous media or stomach, and suitable strength of the preparation.

The orally disintegration time (the time for healthy male or female adults to complete disintegration by buccal saliva) is usually from about 20 to 120 seconds, more preferably from 60 to 90 seconds, and it depends on the amount and type of disintegrant used.

All tablet hardness of the invention were measured with a Schleuniger Tablet Tester 8M apparatus. The hardness of the disintegrable dosage form of the invention is usually from 4 to 6 Kp and the friability of about 1%.

The present invention will be described in more detail by way of examples which should not be construed as limiting the present invention.

### EXAMPLES

### Example 1: Lansoprazole pellets (30 mg dose)

Lansoprazole pellets where prepared by mixing the components in the proportions shown in Table 1:

**Table 1**

| **Quantity (mg)** | **Description** |
|---|---|
| 111,606 | Pellets core |
| 139,294 | Lansoprazole |
| 24,334 | Hydroxypropyl methylcellulose |
| 40,536 | Magnesium carbonate |
| 18,235 | Crospovidone |
| 5,265 | Talc |
| 66,842 | Hydroxypropyl methylcellulose |
| 8,103 | Titanium dioxide |
| 8,508 | Talc |
| 4,050 | Croscarmellose sodium |
| 106,95 | Methacrilic acid - ethyl acrylate copolymer (1:1) dispersion 30% |
| 15,184 | Talc |
| 15,954 | Triethyl citrate |
| 105,711 | PEG 4000 |
| 75,217 | PEG 6000 |
| 193,939 | PEG 8000 |
| 40,658 | Glyceryl monostearate |
| 0,305 | Ferric oxide |
| 6,099 | Saccharin sodium |
| 10,165 | Strawberry flavour |
| 3,049 | Polysorbate 80 |
| **1000.0** | **TOTAL** |

### Examples 2-3: Compression base for lansoprazole ODT - disintegrants

Two lansoprazole orally fast disintegrable tablets were prepared with the compression bases shown in Table 2, with different disintegrants (Crospovidone XL and Starch 1500 - 7%). A hardness range between 4 and 6 kp was studied and all tablets showed a friability about 1% and *in-vivo* disintegration time of about 1 minute in the whole hardness range.

**Table 2**

| **Excipients** | **Ex. 2 (%)** | **Ex 3. (%)** |
|---|---|---|
| Lansoprazole Pellets | 26,93 | 26,93 |
| Xylitol 100 | 62,29 | 62,29 |
| Starch 1500 | 7,00 | - |
| Crospovidone XL | - | 7,00 |
| Aspartame | 2,12 | 2,13 |
| Strawberry flavour | 0,38 | 0,38 |
| Masking | 0,15 | 0,15 |
| Red colorant | 0,01 | 0,01 |
| Sodium stearyl fumarate | 1,13 | 1,13 |
| **Total** | **100,0** | **100,0** |

### Examples 4-6: Compression base for lansoprazole ODT - disintegrants

Three lansoprazole orally fast disintegrable tablets were prepared with the compression bases shown in Table 3, with different fillers (xylitol, mannitol and/or calcium silicate - approximately 63.4%). A hardness range between 4 and 6 kp was studied and all tablets showed a friability about 1% and in-vivo disintegration time of about 1 minute in the whole hardness range.

**Table 3**

| **Excipients** | ***Ex. 4 (%)*** | ***Ex 5. (%)*** | ***Ex 6. (%)*** |
|---|---|---|---|
| Lansoprazole Pellets | 26,9 | 26,9 | 26,9 |
| Xylitol 100 | 63.4 | - | 61,23 |
| Mannitol SD | - | 63.4 | - |
| Calcium silicate | - | - | 2,13 |
| Crospovidone | 7,00 | 7,00 | 7,00 |
| Sodium saccharine | 1,06 | 1,06 | 1,06 |
| Strawberry flavour | 0,38 | 0,38 | 0,38 |
| Masking | 0,15 | 0,15 | 0,15 |
| Red colorant | 0,01 | 0,01 | 0,01 |
| Sodium stearyl fumarate | 1.13 | 1.13 | 1.13 |
| **Total** | **100** | **100** | **100** |

## Claims

1. Orally fast disintegrable pharmaceutically acceptable multiple units tablet dosage form comprising a) tablet excipients comprising a disintegrant and b) individual units wherein each individual unit comprises:
i) a core material comprising at least one physiologically active substance;
ii) a controlled release coating layer; and
iii) an over-coating layer comprising a mixture of plasticizer agents.

2. Dosage form according to claim 1, wherein the over-coating layer comprises a mixture of a first polyethylene glycol and a second plasticizer agent.

3. Dosage form according to claim 2, wherein said second plasticizer agent is a second polyethylene glycol.

4. Dosage form according to claims 2 or 3, wherein the mixture of plasticizer agents comprises a polyethylene glycol with an average molecular weight lower than 6000.

5. Dosage form according to claim 4, wherein the over-coating layer comprises a mixture of polyethylene glycol 4000 and polyethylene glycol 6000 as plasticizer agents.

6. Dosage form according to claim 5, wherein the plasticizer agent comprises a mixture of polyethylene glycol 4000, polyethylene glycol 6000 and polyethylene glycol 8000.

7. Dosage form according to any of claims 1-to 6, wherein the controlled release coating layer is an enteric coating layer.

8. Dosage form according to any of claims 1 to 7, wherein the over-coating layer comprises more than 80% w/w of a plasticizer mixture.

9. Dosage form according to claim 8, wherein the over-coating layer comprises more than 90% w/w of a plasticizer mixture.

10. Dosage form according to claim 9, wherein the over-coating layer comprises more than 95% w/w of a plasticizer mixture.

11. Dosage form according to any of claims 1 to 10, wherein the at least one physiologically active substance is an acid labile substance.

12. Dosage form according to claim 11, wherein the acid labile substance is an H+/K+-ATPase inhibitor.

13. Dosage form according to claim 12, wherein the acid-labile H+K+-ATPase inhibitor is a benzimidazole derivative or one of its single enantiomers or a salt thereof.

14. Dosage form according to claim 13 wherein the benzimidazole derivative is selected from the group consisting of omeprazole, pantoprazole, rabeprazole, lansoprazole or one of its single enantiomers or a salt thereof.

15. Dosage form according to any of claims 11 to 14, wherein the acid-labile substance is mixed with alkaline compounds.

16. Dosage form according to any of claims 1 to 15, which further comprises one or more separating layer(s) over the core material and below the controlled release coating layer.
